# EUROPEAN PATENT APPLICATION

(11) **EP 2 128 618 A1**
(43) Date of publication of application: **02.12.2009**
(21) Application number: 08157297.6
(22) Date of filing: 30.05.2008
(51) Int. Cl.: G01N 33/543, G01N 33/68

(54) **A process for monitoring the presence of pathogenic agents in biological fluids by nanostructured materials**

(71) Applicant: Prion Diagnostica S.r.l., 20017 Rho (IT)
(72) Inventor: Balzano, Maria Francesca, 07040 Stintino (IT); Basagni, Massimo, 20020 Arese (IT); Fresu, Sonia, 07041 Alghero (IT); Innocenzi, Plinio, 07041 Alghero (IT)
(74) Representative: Gervasi, Gemma

(57) **Abstract**

This invention relates to the use of a nanostructured material selected in the group consisting of: oxides of transition metals, mixed oxides of transition metals and their mixtures to detect the presence of contamination by the pathological isoform of the prion protein (PrPsc) in a sample.

## Description

### Field of the invention

This invention relates to the use of nanostructured materials for in vitro diagnostics of pathogens made up by prionic proteins that can contaminate and/or infect ruminants (sheep, cattle, etc..) and other mammals, by their localization in tissues and fluids destined also to human consumption.

This invention is also relevant to the synthesis of hybrid organic-inorganic materials as biosensors for selective capturing and detecting of the pathological form of the prion protein. More specifically these materials are functionalized with molecules having affinity both specific and not specific, showing higher ability to catch (and / or discriminate).

### State of the art

The Prion protein (PrP) is a hydrophobic protein of 27-35 KD, codified by the gene PRNP, quite common in the organism of various animal species. It is present in mammals - and particularly in ruminants - but is found also in certain fish (such as salmon) and other organisms.

It is well known that this protein has the characteristic of being able to change the spatial conformation of the molecule, while maintaining the same chemical formula, in circumstances not well clarified so far. This transformation from the "alpha-helix" structure to the "beta-sheets" involves the onset of infectivity characteristics of the protein itself, with pathological consequences for the organisms with which in various ways the protein comes in contact.

The prion protein with "normal" structure is usually indicated as PrPc, while the modified structure (beta-sheet) responsible for several neurodegenerative diseases is listed as PrPsc and is often called Prion.

Another important fact is the ability of the PrPsc to induce the transformation of the PrPc protein into the PrPsc, where the two protein forms are contemporary present in the body (for example in various tissues and / or biological fluids).

It should also be noted that there is a serious difficulty in distinguishing the two forms of prion protein potentially present in tissues and / or organic fluids - as well as in soil and the environment - that makes highly complex and uncertain the recognition for diagnosis and prevention. This is particularly worrying if one takes into account that proved the ability of prion "trigger" the transformation process PrPc → PrPsc, with the development of infectivity in the body, even starting from very low concentrations of form "infectious" PrPsc in an organic tissue, so it is extremely important to have methods capable of facing the problem of prion detection in tissues and fluids with very small quantities. The prion diseases are known since long time (from BSE or "mad cow disease", to Scrapie diffused among sheep and possible "door way" for BSE).

Until few years ago it was thought that for effective protection of human health to these dangerous diseases was sufficient to avoid the entering into the human food chain of certain mammals tissues, such as the brain, the lymph nodes, the bone marrow, etc.. (known as SRM = specific risky material).

Today there is the suspect that the ways of prion infection may be more numerous and "subtle"; for example, it is feared that prions may move into the flow of fluids such as blood, milk, urine; fluids that, in different ways, could easily contaminate both the environment and - directly or indirectly - the consumer.

A more detailed picture of the epidemiological situation is showed below.

As it is well known, the most widespread prion disease - the Scrapie - belongs to a group of neurodegenerative diseases, transmissible and fatal, that may affect both the man and animals, indicated by the term Transmissible Spongiform Encephalopathies (TSE). To date, different forms are known, including the Creutzfeldt-Jakob disease (CJD), his youth variant (vCJD), the Gerstmann-Sträussler-Scheinker (GSS) and the Fatal Familial Insomnia (FFI) which affect humans. Among animal TSE, the most notable, in addition to Scrapie, are Bovine Spongiform Encephalopathy (BSE) in cattle, Feline Spongiform Encephalopathy (FSE) in cats and other Scrapie-like forms affecting wild ruminants. The typical lesions of these diseases are localized only in the central nervous system (CNS) where it has been observed gradual vacuolation of the dendritic and assonal processes and of the cell bodies of neurons, diffused astroglial hypertrophy and proliferation, widespread spongiform deterioration of the grey matter accompanied by extensive neuronal degeneration and intra- and extra-cellular deposits of amyloid substance. Currently, the most accredited etiological hypothesis, proposed by Prusiner, identifies the pathogen with a prion, that is the pathological isoform (PrPres = PrPsc) of a protein normally produced at the cellular level (PrPsen = PrPc).

Many years after the appearance in the UK of the first case of bovine spongiform encephalopathy (BSE), it has made its appearance the variant Creutzfeldt-Jacob Disease (vCJD) related to BSE, raising the important issue of the identification of the source of contamination for humans [Almond J. & J. Pattison (1997); Will RG. (1996)]. Concerning cattle infected with BSE we witnessed a shutdown of the BSE epidemic in the UK with the elimination of animal-derived flours from the diet of these animals; it is therefore likely that the source of contamination of the cattle in this country has been eliminated completely or at least reduced to very low levels.

Nevertheless, the theoretical and difficult to quantify risk remains, that BSE is present in the sheep population, given that the UK animal meal contaminated with BSE were probably used to feed small ruminants and that BSE was experimentally transmitted to sheep through the oral route with quantities of only 0.5 grams of infected brain [Foster JD et al, 1993; Foster JD. et al., 2001 st Foster JD. et al., 2001 b].

BSE in sheep is indistinguishable from Scrapie from a clinical point of view and, at pathogenetic level, the BSE in the ovine is characterized by the involvement of the lymphoreticular system. Moreover, the BSE like the Scrapie, can be transmitted to the ovine through the transfusion of whole blood taken from another sheep during the pre-clinical trial of infection with BSE, when the donor appears clinically healthy [Hunter N. & F. Houston, 2002; Hunter N. et al, 2002]. Therefore, the possibility that sheep have been fed food contaminated with BSE raises the real issue that BSE could be present in the sheep population without any possibility of distinguishing it from Scrapie. It is also likely that the BSE agent is transmitted within the sheep population, as is the case for Scrapie, by vertical and horizontal transmission, thus creating a new source of exposure to BSE to humans [Crozet C. al, 2001]. In order to assess the risk to humans would be essential to carry out a differential diagnosis, but at present there are no rapid tests that can distinguish BSE from Scrapie (Scientific Steering Committee, 2001). Although currently is not clear if BSE is present in ovicaprines in the field and even if since 2001 did not appear to exist indications of an increase in the incidence of TSE cases in small ruminants, the presence of BSE in sheep can not yet be excluding (Scientific

Steering Committee, 2001).

In view of the scarce reports of TSE cases in small ruminants in Europe, which are not enough to highlight small changes in terms of incidence, and in order to obtain reliable information on the prevalence of these diseases even in the ovicaprines, national programs of surveillance of TSE in small ruminants began in EU, based on the implementation of rapid tests, from January 2002. Currently, at the level of

European Community, detailed provisions are in force (EC Regulation No. 99/2001) which require the implementation of rapid tests for TSE positivity on a representative sample of the ovicaprines population. The legislation also plans that, for those subjects resulted positive to the rapid test for Scrapie, it has to be performed a molecular analysis to evaluate the genotype on the prion protein (PrP).

So, Scrapie is a potential risk characterized by at least two very serious components, namely:
I. Risk for the spread of the (lethal) disease among sheep (and / or goats);
II. Risk for the possibility of transmitting BSE, by mistaking it with Scrapie, among sheep and goat and then from these animals to humans.

Considering what stated, it is evident that just the Scrapie disease of the ovicaprines constitutes a serious risk both to all livestock and humans. That risk threatens the production of the very important Agriculture Industrial sector that is the sheep farming and the induct of the lacto-dairy products, with its strong export component. Especially strong is the impact, both economical and environmental, that this disease exerts on specific territories. The emergence of repeated outbreaks constitutes a serious threat to the entire territory affected; in fact, the event of Scrapie outbreaks determines, among others, to the necessary destruction of entire flocks (with the consequent need their incineration and disposal of ashes) and to increasing modification of the eco-environmental balance of the territory and its specificities.

Considering the economic importance of the ovicaprine sector, in Italy and many countries in the world, it is well understood the importance of developing new methods to improve the programs for Scrapie monitoring and control. To date it does not exist a specific instrument of intervention to resolve the problems cited above and, especially to tackle the problem of a possible " Scrapie crisis." However it seems reasonably possible, using innovative technologies, to develop relatively quickly potentially effective instruments.

Given the substantial structural homology between different prion "strains" in the different pathological forms, these instruments could be applied, of course, also for controlling other TSEs in other species of animals and humans, and - presumably - even for controlling other human neurodegenerative diseases.

The development of possible tests for Scrapie and other TSEs early detection, and also possibly for the so-called " *in vivo* diagnosis," is particularly useful at the moment. In fact, the direct detection of prions, considered the most reasonable and appropriate approach to diagnose and monitor the onset and evolution - both horizontally and vertically - of prion diseases, has given rise to many techniques aimed at detecting the presence of the modified prion protein (PrPsc) in mammalian tissues. In particular, real "routine tests" were developed by various research laboratories and companies, some of these tests have obtained the "validation" by the European Union to be marketed and used on a large scale on slaughtered animal. All these tests are not for now able to detect the presence of PrPsc in biological fluids (blood, milk, urine), and then they do not fit for early diagnosis (and in any case, "*in vivo*") of Scrapie and other TSE.

Currently, a technique widely used for the analysis of the prion protein is identified with the Western Blotting (WB) which consists of an immunodiagnostic method associated with the chemiluminescence revelation (ECL - Enhanced Chemiluminescence) characterized by high sensitivity that can ensure detection of small quantities of specific antigens.

Other techniques - also of immunodiagnostic type - were developed applying the ELISA approach. All these techniques have proven, at practical tests, useful for the detection of prions in tissues containing PrPsc concentrations high enough and - above all - very homogeneous, but have proven not to be effective for "in vivo" diagnostics based on the analysis of biological fluids where the PrPsc may be present at very low concentrations and, above all, in a inhomogeneous way.

Therefore the problem of the development of assays with enough sensitivity that can also be used on fluids remains open, and for detecting the pathogenic protein even in living individuals and / or at a very early stage of development of the disease.

It is known that the spatial structure of prion protein is different in the two isoforms PrPc and PrPsc, while maintaining the same chemical formula and although these isoforms are equally "recognized" by antibodies anti-PrP. However, the immunological tests based on antibodies exploit, in order to reveal the presence of PrPsc in biological samples, a further property of PrPsc, that is to be significantly resistant to enzyme digestion processes, for example by the Proteinase K (PK).

In an effort to develop diagnostic tests sufficiently sensitive for PrPsc, the binding properties of various molecules in addition to natural antibodies, such as heparin, glycosaminoglycans, Congo Red, quiinacrin and some metals (copper, manganese) (see Touil et al . J. Pharm. 2006 40:822-832), and binders or "synthetic" peptides, such as the dendrimers (Solassol et al., 2004, J. Gen. Virol., 85, 1791-1799, Lau et al., 2007, PNAS, 104, 11551-11556), which however are not discriminatory for the pathological isoform of prion protein.

Moreover, it was also described the ability of the sodium salt of phosphotungstic acid to bind / precipitate the PrPsc isoform under specific conditions (JDF Wadsworth et al. The Lancet 2001, 358: 171).

Finally, it is known that proteins, eg. cytochrome C, can be immobilized with high affinity on molecular sieves of mesoporous material (Washmon-Kriel et al. J. Mol. Catalysis 2000, 10:453-469).

### Summary of the invention

This invention relates, according to a first aspect, to a system for selective capture and detection by a nanostructured material selected in the group consisting of: oxides of transition metals, mixed-oxides of transition metals and their mixtures, of the pathological isoform of the prion protein (PrPsc) in an unknown sample. Especially preferred is the nanostructured material selected in the group consisting of: silicon oxide, titanium oxide, mixed oxides of silicon and titanium and / or silicon and / or zirconium (SiO₂-ZrO₂, SiO₂-TiO₂) and is preferably in the mesoporous form with a porosity ranging between 2 and 50 nm. Even more preferably the material is silica or titania and is placed as a film on a suitable support. Alternatively is in the form of nanoparticles. Increased efficiency / standardized detection of prion protein is obtained by functionalizing the surface of the film with amino groups. Even higher is the responsiveness of the film after further post-functionalization with a molecule having affinity for the prion protein, selected in the group consisting of: polyoxometallates (POM), Congo Red, glycosaminoglycans, heparin or their mixtures.

The detection of the selective link of the pathological isoform of the prion protein mainly depends on the type of support used and is preferably obtained by a method selected in the group consisting of: FT-IR (Fourier Transform Infrared spectroscopy), NMR (nuclear magnetic resonance), XRD (Powder X ray diffraction), SPR (Surface Plasmon Resonance), UV-Vis (UltraViolet-Visible), HP (Cyclic Voltammetry).

In particular if the detection is made through FTIR, the preferred wavelength range is comprised from: 3800-400 W cm⁻¹ and even more preferably in the following sub-ranges : 3800-3200 W cm⁻¹, 1750 -1350 W cm⁻¹ and 1000-400 W cm⁻¹. By this analytical method, the signal detected with an unknown sample is compared for at least one of the following characteristics: intensity of absorbance, area under the curve, shape of the curve and / or translation (shifting) of vibration frequencies.

The sample is a biological sample or a food sample i.e. a tissue homogenate or milk, plasma or other. Preferably the sample consists of milk, even more preferably from ovicaprines population, even suffering from Scrapie.

According to a further aspect, the invention relates to a process for the preparation of a support coated with a nanostructured material, wherein such material is a transition metal oxide selected among mesoporous silica and titania, with porosity ranging between 2 and 50 nm, characterized for comprising the following steps:
a. preparing a sol-gel from a silicon or titanium alcoxide through hydrolysis in the presence of a polar solvent,
b. mixing of the sol-gel with a solution containing a surfactant (ionic, preferably cetyl-trimethyl-ammonium-bromide, or non-ionic, preferably a block copolymer) dissolved in a polar solvent,
c. synthesizing a film from the above mentioned mixture on a suitable substrate by one of the following techniques:
   - immersion and extraction at controlled speed ( "dip-coating")
   - deposition by centrifugation eg. on a substrate in rotation at a controlled speed ( "spin-coating")
   - by spraying ( "spray-coating")
d. densificating the film by drying and / or heating at temperatures above 300 ° C, preferably air at a temperature of about 500 ° C;
e. functionalizing the film with a molecule having amino groups, in a polar solvent;
f. further functionalizing the densified and functionalized film with a material having affinity for the prion protein through a suitable functional group.

Preferably, in step e) the the amino group bearing molecule is aminopropyltriethoxysilane (APTES) or aminoethyl-aminopropyltriethoxysilane (AEAPTES).

According to a further aspect, the invention relates to supports of various kinds, preferably silicon or quartz obtained by this procedure, coated with a film of mesoporous nanostructured material functionalized with a molecule having amino-groups and then with a molecule having affinity for the prion protein, such as a polyoxymetallates, preferably NaPTA (sodium salt of the phosphotungstic acid), Congo Red, glycosaminoglycans and heparin.

### Brief description of figures

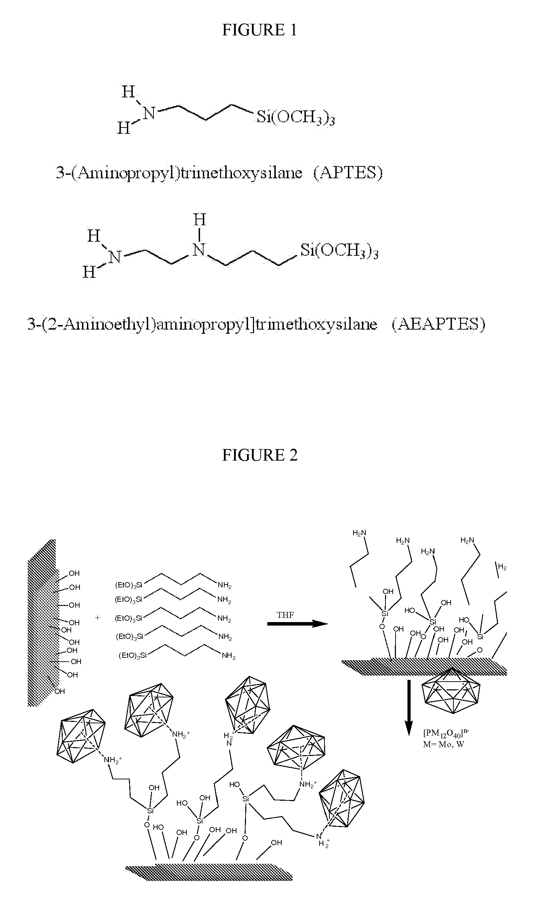

**Figure 1****.** Schematics of modified alcoxides bearing amino groups (APTES and AEAPTES) useful for the functionalization of mesoporous or dense surfaces.
**Figure 2****.** Diagram showing a procedure for preparing the hybrid organic-inorganic material according to one embodiment of this invention.

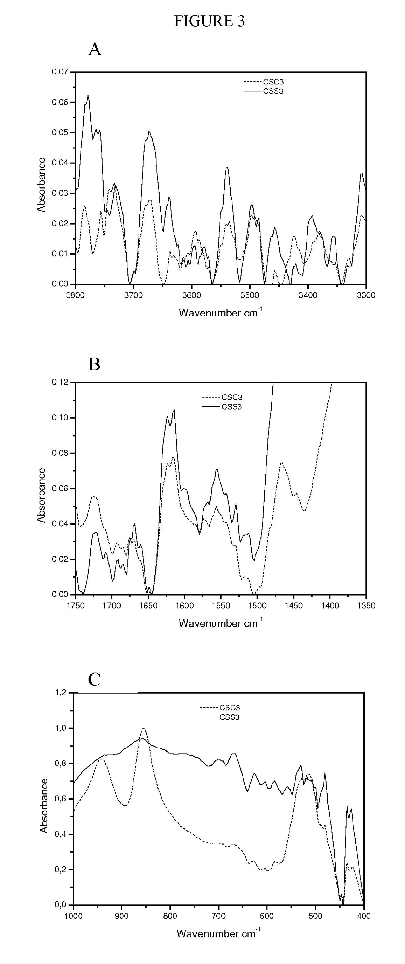

**Figure 3****.** FTIR absorption spectra assessed in the 3800-400 W cm⁻¹ range, divided in the intervals 3800-3300 (panel A), 1750-1350 (panel B) and 1000-400 (panel C) W cm⁻¹, obtained incubating mesoporous films functionalized with APTES (MS-APTES) in samples of Scrapie positive (S3) and negative (C3) brain homogenates.

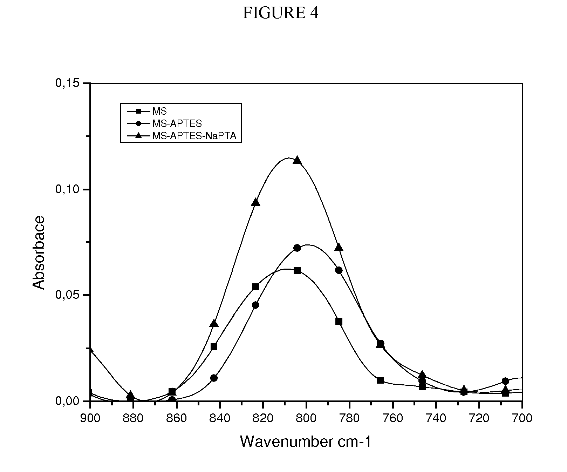

**Figure 4****.** FTIR absorption spectra on a defined range of wavelength (900-650 W cm⁻¹) of a film deposited on a mesoporous substrate during different stages of preparation: 1. - v - mesoporous film (MS), 2. - λ - mesoporous film whose surface was functionalized with aminopropyletoxisilane (MS-APTES), 3. - σ- mesoporous film whose surface was functionalized with an aminopropyletoxisilane binder and a polyoxometallate natural binder (MS-APTES-POM).

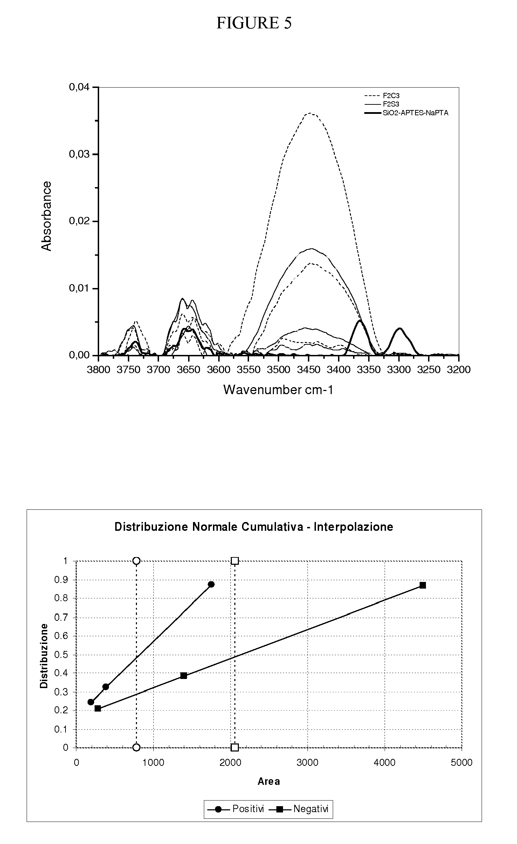

**Figure 5****.** FTIR absorption spectrum in the 3800-3200 W cm-¹ wavelength range of 6 samples deposited on mesoporous film (MS-APTES-POM, used only as example) incubated in brain positive (F2S3) and negative (F2C3) samples, each of which is divided into three replicas; the spectrum shows a systematic different absorbance value between positive and negative samples. The chart below (panel B) shows the cumulative normal distribution areas under the curves produced by the positive (F2S3) and negative (F2C3) samples corresponding to the spectrum shown (see also Table 1).

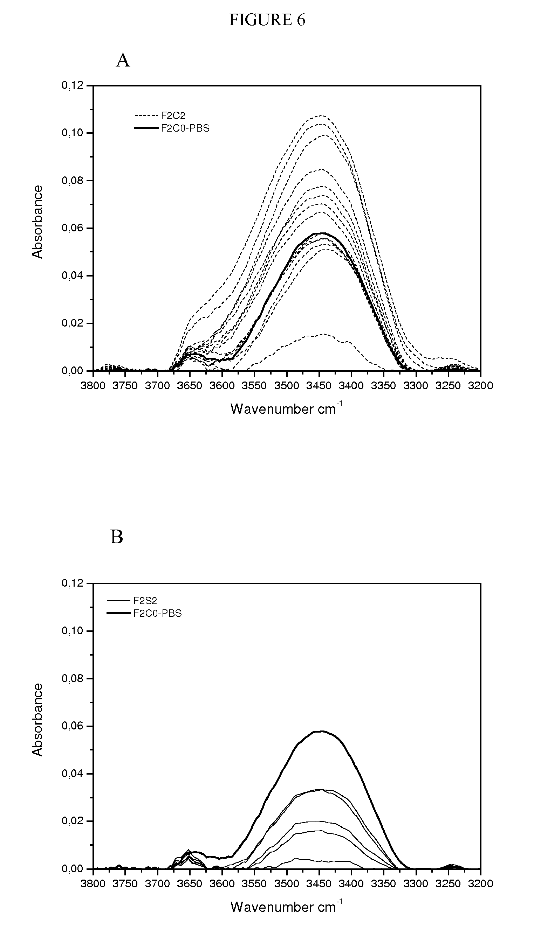

**Figure 6****.** FTIR absorption spectra, in the 3800-3200 W cm⁻¹ range, obtained from homogenate brain samples of sheep, either (N = 5) clinically positive Scrapie (marked with the initials "S2", panel B) or (other N = 15 samples) clinically negative (marked with the initials "C2", panel A) deposited on mesoporous film (MS-APTES-POM) and incubated in homogenate brain (20%) in 10 ml of PBS 1x. In this range it is particularly evident the systematic difference between absorbance curves produced by negative and positive samples. The spectrum obtained with the incubation of the film into the buffer only (PBS) is the discriminatory element of positive and negative spectra.

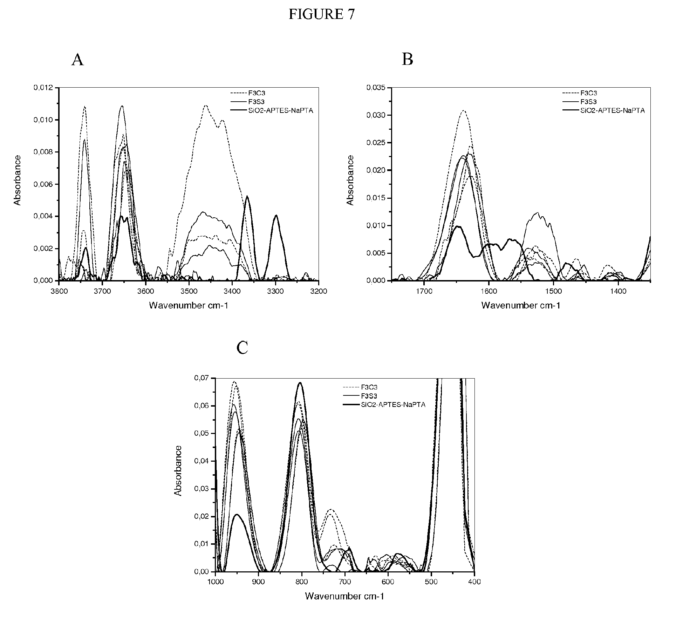

**Figure 7****.** The figure, with table below, shows the results of the changes in FTIR spectra obtained from samples deposited on mesoporous film (MS-APTES-POM) incubated into 20% homogenate brain (3 clinically negative samples: C3R1, C3R2, C3R3 and 3 samples clinically positive S3R1, S3R2, S3R3), considering the difference between the absorbance values before (test F2) and after samples digestion with a 10% proteinase K solution (test F3). It is shown, as indicated in the table below, a maximum height variation of the curve ("peak fall") which is regularly more significant for the negative samples compared to the positive in the three wavelength intervals used (panels A, B, C).

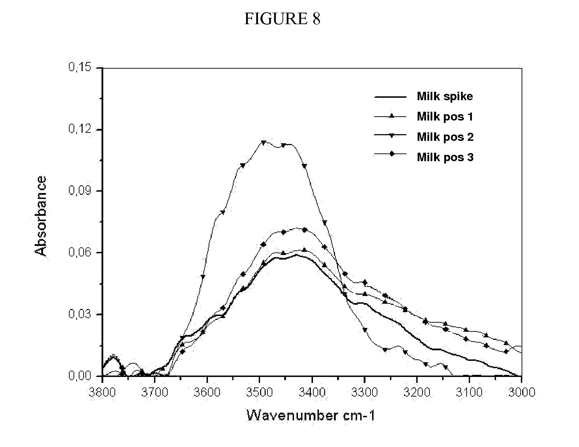

**Figure 8****.** FITR absorption spectra, assessed in the 3800-3000 W cm⁻¹ range, of mesoporous films obtained according to the invention and tested with 3 sheep milk samples (-■- , -●-, -▲-).
   The FTIR spectrum of a sample, obtained by adding PrPsc in the milk of a healthy animal through inoculation ("spiking") into the milk of a brain homogenate obtained from an animal suffering from Scrapie (spike milk), is compared to the spectra obtained from milk samples of various entities, in which milk it cannot be excluded the presence of PrPc / PrPsc, as Scrapie-positive sheep lactating, belonging to a group of sheep subjected to an experimental infection by brain inoculation of a Scrapie strain. The sample Pos 1 came from a individual whose breast tissue resulted positive to official tests for the presence of PrPsc and who suffered from chronic mastitis.

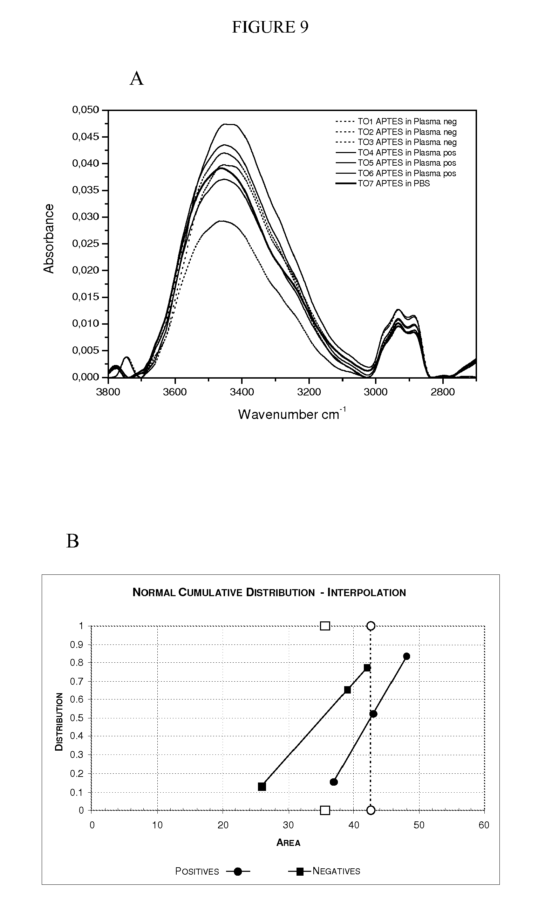

**Figure 9****.** FTIR absorption spectra in the 3800-2700 W cm⁻¹ range of mesoporous film functionalized with APTES (TO APTES) incubated into positive and negative plasma samples. T07 APTES in PBS refers to a sample of mesoporous titania films incubated into PBS. The table and graph below show the normal distribution of both areas under the curves produced by the negative samples for PrPsc (TiO1-2-3 APTES in Plasma neg) and positive (T04-5-6 APTES in Plasma pos) referred to the spectrum shown in Figure 9.

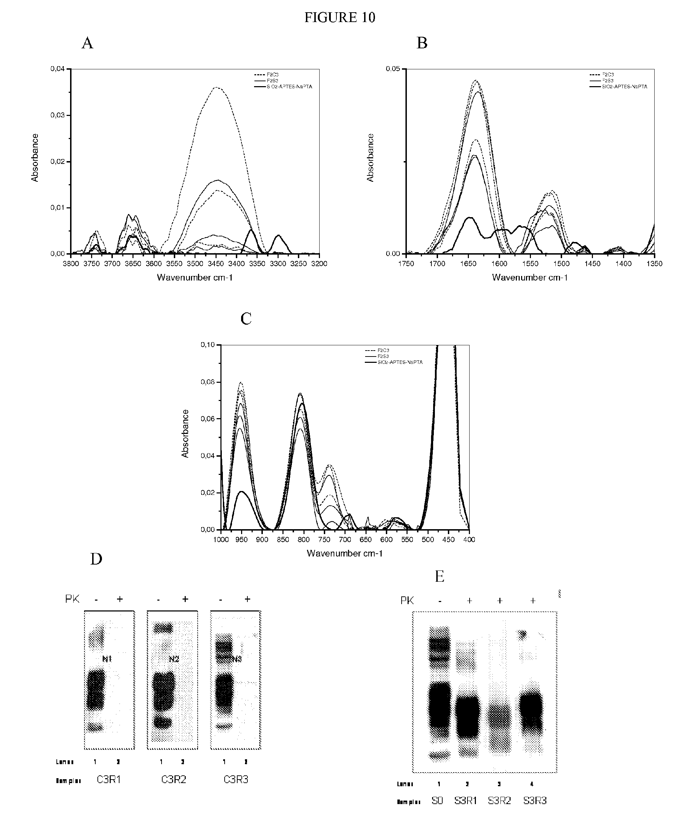

**Figure 10****.** FTIR absorption spectra in the 3800-3200 W cm⁻¹, 1750-1350 W cm⁻¹ and 1000-400 W cm⁻¹ ranges (panels A, B, C), of mesoporous film (MS-APTES-POM) incubated into PrPsc positive (S3) and negative (C3) brain samples, each of which divided into three replicates (R1, R2 and R3). The results obtained are compared with those obtained on the same samples using the reference procedure "Western Blotting" (panels D, E), which is considered "gold standard" for the official control analysis of the two prion protein isoforms PrPc and PrPsc, carried out on homogenate brain samples taken from slaughtered animals (cattle /sheep). Panel D: negative controls (C3), lanes 1 and 2: digested or not with PK; panel E: 3 positive samples (S3): digested or not with PK (proteinase K).

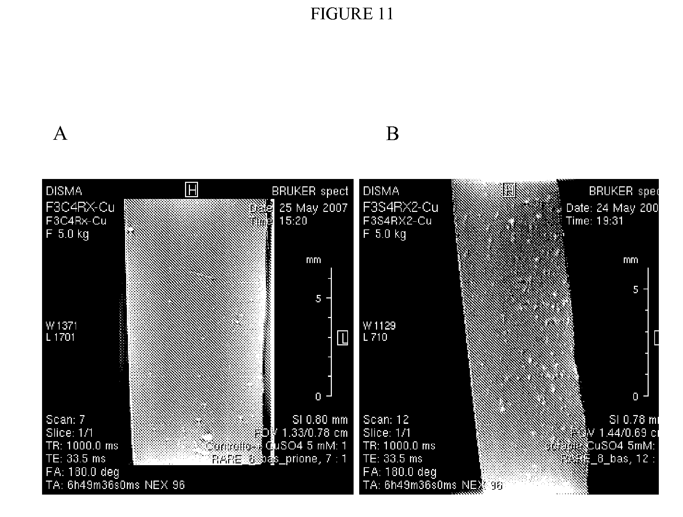

**Figure 11****.** Micro-imaging NMR of mesoporous films (MS-APTES-POM) obtained according to the invention and incubated into Scrapie positive or negative homogenate brain samples, in which it is evident a level of perturbation much more intense in the case of positive samples (containing PrPsc) compared to negative samples. Panel A: Scrapie negative sample; panel B: Scrapie positive sample.

### Detailed description of the invention

### Definitions:

Nanostructured material: it defines a nanostructured system made up of groups of atoms or molecules with an average size ranging from 1 to 100 nanometers.

POM: polyoxometallates.

FTIR: Fourier transformed infra-red spectroscopy.

Transmissible Spongiform Encephalopathies (TSEs). TSEs are neurodegenerative diseases, transmissible and fatal, that may affect both man and animals. This term means different forms of neurodegenerative diseases, eg. The Creutzfeldt-Jakob disease (CJD), his youth variant (vCJD), the Gerstmann-Straussler-Scheinker (GSS) and the Fatal Familial Insomnia (FFI) which affect humans. Among the animal TSEs, the most widespread prion disease is the Scrapie. The best known, in addition to Scrapie, are the Bovine Spongiform Encephalopathy (BSE) in cattle, the Feline Spongiform Encephalopathy (ESF) of cats and all the other Scrapie-like forms affecting wild ruminants. The variant of the Creutzfeldt-Jakob Disease (vCJD) related to BSE, has made its appearance several years after the appearance of the first case of bovine spongiform encephalopathy (BSE) in the UK.

In the ovine, BSE is indistinguishable from Scrapie from a clinical point of view and, at pathogenetic level, the BSE in the ovine is characterized by the involvement of the lymphoreticular system. Moreover, the BSE like the Scrapie, can be transmitted to the ovine through the transfusion of whole blood taken from another sheep during the pre-clinical trial of infection with BSE, when the donor appears clinically healthy [Hunter N. & F. Houston, 2002; Hunter N. et al, 2002]. All TSEs are caused by accumulation of amyloid substance generated as a result of structural changes in the central nervous system caused by transmissible pathogens.

Prion. According to the most accredited etiological hypothesis proposed by Prusiner, the prion is the agent of TSEs and corresponds to the pathologic isoform (PrPres = PrPsc) of a protein normally produced at the cellular level (PrPsen = PrPc). It is known that the spatial structure of prion protein is different in the two isoforms PrPc and PrPsc, while maintaining the same chemical formula and although these isoforms are equally "recognized" by antibodies anti-PrP. However, the immunological tests based on antibodies are based, in order to reveal the presence of PrPsc in biological samples, on a further property of PrPsc, namely to be significantly resistant to enzyme digestion processes, for example by the Proteinase K (PK).

### Description

It is known that nanotechnology will permit to control the intimate structure of materials and their interactions with biological molecules on a nanoscale. On this scale (typically below 100 nm) the physicals-chemicals properties of the materials and of their interaction is different from that of bulk materials ( "bulk") and new properties arise. It is possible to use these new properties to develop innovative systems for applications in various fields, particularly in biotechnology where it is necessary to have systems for the selective capture and / or detection of specific organic molecules, particularly when these may be associated with human or animal diseases.

During some tests for the surface functionalization of nanostructured materials (systems formed by groups of atoms or molecules with an average size ranging from 1 to 100 nanometers; at this scale of dimensions entirely new phenomena and physical-chemical behaviors can be observed) aimed at assessing the stability, quality and regularity of functionalization of materials through reading of FTIR spectra, after incubation of such materials in various chemicals or into solutions derived from biological materials, it has been surprisingly observed that the spectra characteristics (values of absorbance and patterns in different spectral wavelength range) varied after exposure of such materials to samples containing different structural forms of the prion protein.

It has been verified that this selective adsorption is reproducible with different nanostructured materials, either made of simple and / or mixed transition metal oxides synthesized in the form of films. The material is preferably in the mesoporous form, preferably with a porosity comprised from 2 to 50 nm.

The nanostructured material is also preferably distributed as a film on an inert support; the selective adsorption of the pathological isoforms on the film is detected through detection methods that depend on the support on which the film was synthesized. In particular, FTIR spectroscopy is used if the support is silicon-made, UV-Vis in case the support is quartz, as known to the skilled artisan working in the field.

Preferably the nanostructured material is silica or titania. Alternatively to the shape of film, the nanostructured material is realized in the form of nanoparticles.

The detection of a different interaction between the film exposed to biological samples containing or not the pathological isoform of the prion protein is carried out, according to the preferred embodiment of the films, by vibrational spectroscopy techniques such as infrared spectroscopy (FTIR). The results obtained, on mesoporous films exposed to different biological materials, are surprising for selectivity and high reproducibility and can be also highlighted other techniques, such as NMR (nuclear magnetic resonance), the SPR (surface plasmon resonance) and UV-Vis.

The mesoporous materials have a controllable porosity (in the range of 2-50 nm) topologically distributed and ordered, which is obtained by using a surfactant that can be ionic, like the cetyl-trimethyl-ammonium-bromide (Grosso et al., Chem. Mater. 2002, 14, 931-939) or non-ionic, such as block copolymers (Kipkemboi et al., Langmuir 2001, 17, 5398-5402), dissolved in a polar solvent and water. One of the advantages of using such materials is inherently due to the high surface area and thus the possibility of having a large number of functional groups (preferably amino-groups) on the surface. The dense films have a not controlled porosity and, despite having the same responsiveness of mesoporous films (in terms of functional groups), are mainly reactive at a superficial level. They are prepared by using the same process to obtain mesoporous films, but in the absence of surfactants.

According to a preferred aspect, the surface of films or nanoparticles is further derivatized with amine functional groups, preferably with an organically modified alcoxide carrying amino groups.

It has been also observed that when the surface of these materials undergoes a further post-functionalization with molecules having affinity for the prion protein, although not necessarily having specific affinity for the pathological isoform and particularly if these molecules are preferably selected in the group consisting of polyoxymetallates, Congo Red, glycosaminoglycans and heparin, it is possible to obtain a product where the sensitivity to the selective adsorbance of the variant of the prion protein structurally related to the pathological isoform is further increased.

Thus the invention further comprises the use of:
- support of a inert material coated with a film of a nanostructured material made by a simple oxide and / or a mixed oxide of a transition metal, preferably mesoporous,
- support whereon this film is further derivatized with amino groups and
- support whereon the film, functionalized with amino groups, is further post-functionalized with molecules having affinity for the prion protein,
to detect the contamination of a sample containing the pathological isoform of prion protein.

Particularly preferred for the detection of support coated by films where the film is mesoporous silica, is the detection by FTIR.

The reading of a layer covered with a film of mesoporous nanostructured material, incubated in a biological sample, by FTIR allows to highlight the presence of several structural components of molecular species captured by the film, are recognizable by " specific frequencies" of vibration. The wavelength ranges where preferential differences (in terms of absorbance, area under the curve, shape and shift of vibration frequency), corresponding to the interaction of the film with samples containing or not the pathological isoform PrPsc, have been found are those included in the following sub-ranges: 3800-3200 W cm⁻¹, 1750-1350 W cm⁻¹ and 1000-400 W cm⁻¹.

If the detection is made through FTIR reading, the wavelength intervals where the differences (in terms of absorbance, area under the curve, shape and shift of vibration frequency), corresponding to the interaction of the film with samples containing or not the pathological isoform PrPsc, are in particular the following three: 3800-3200 W cm⁻¹, 1750-1350 W cm⁻¹ and 1000-400 W cm⁻¹, analyzed either simultaneously or alternatively one to the other. Preferably, the difference in area underlying the curve is measured.

According to a preferred aspect, the invention also comprises a step of preparation of a standard negative reference sample, certainly free of protein in its pathological form (PrPc). The sample is therefore a homogenate or a biological liquid coming from herds certified as negative by any of the methods available to date for certification under the EC Regulation N.260/2005, February 16, 2005, prepared in the same way as the unknown sample, namely with the same diluting solutions, and preferably in the same organic matrix. The methods for the preparation of controls, both positive and negative, are known to the skilled man, as the necessary comparison of signals is essential for the proper interpretation of the results.

The preparation of nanostructured material films suitable for the detection of the isoform of the prion protein according to the invention, and whose surface is preferably derivatized with amine groups and further modified by post-functionalization with polyoxymetallates compounds or other molecules having affinity for the prion protein includes the following steps (suitably modified by the technician in the field):
a) Preparation of a sol starting from simple oxides or mixed oxides of transition metals, preferably from alcoxides of silicon or titanium (typically tetraethoxysilane and similar as a precursor of silica, or titanium tetrachloride and similar as precursor of titania), or different transition metals or their compositions (typically SiO2-ZrO2, SiO2-TiO2) in a solvent.
   Alcohols mixable with water or water-soluble solvents such as tetrahydrofuran (THF), water and a catalyst can be used for hydrolysis reactions and alcoxides condensation. The solution is used for film coating by immersion and extraction at a controlled speed on a suitable inert substrate ("dip-coating") or by deposition of the solution on a substrate in rotation at controlled speed ("spin-coating") or by spraying ("Spray-coating"). According to the preferred embodiment of a mesoporous film material a second solution containing a surfactant is then prepared [ionic as the cetyl-trimethyl-ammonium-bromide (Grosso et al., Chem. Mater. 2002, 14, 931-939) or non-ionic as block copolymers (Kipkemboi et al., Langmuir 2001, 17, 5398-5402)] and dissolved in a polar solvent and water. This solution is mixed to the other and the resulting solution is used for the film-coating.
   According to a preferred embodiment, supports are silicon or quartz wafers. However, any surface (such as polystyrene, polypropylene) , after activation by techniques known from the literature ( 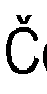 ernáková et al., 2005 Pasma Chem and Plasma Process., 25 (4); Suni et al, 2002, J. Electrochem. Soc., 149, 348; Wiegand et al, 2000, J. Electrochem. Soc., 147, 2734) can be used for nanostructured material synthesis.
   After film coating, preferably having a thickness comprised from 250 to 300 nm, a heat treatment for densification is carried out. The heat treatment is performed using air at temperatures typically around 500 °C.
b) Functionalization of the surface. A second solution containing an organically modified alcoxide with amine functional groups is prepared and the film surface is derivatized. Preferably an organically modified alcoxide with amine functional groups is utilized, typically aminopropyltriethoxysilane (APTES) or aminoethyl-aminopropyltriethoxysilane (AEAPTES). Tetrahydrofuran is used as solvent, but other polar solvents such as alcohol, can be used (Salmio and Brohwiler, J. Phys. Chem. C, 2007, 111, 923-929). The film is then immersed in this solution for a time necessary to implement the reactions of anchoring the alcoxide containing amines on the surface of the porous and non-porous material (see above). Alternatively to this methodology, it is possible to perform a "one-pot" synthesis techniques (Angelome et al, 2005, J. Mater. Chem., 15, 3903-3912) in which the mesoporous film is functionalized with amine groups. The process of functionalization of such areas has shown to be very effective and reproducible.
c) Anchoring (or post-functionalization) of molecules having affinity for the prion protein, optionally suitably modified to increase the compatibility of reaction with the reactive groups on the surface of the film. The anchorage step is preferably made with polyoxometallates (POM) and even more preferably with NaPTA. Anchoring is performed by dipping the porous or solid matrix, whose surface was previously functionalized with amine groups. The POM (or other compounds) are dissolved in THF (tetrahydrofuran) or water and the material is maintained submerged in the solution for some hours until the anchoring process on the film through the amine groups is ended.

Preferably the films are made of nanostructured mesoporous silica (silicon oxide) or titania (titanium oxide), with porosity of between 2 and 50 nm, so their preparation includes, according to a preferred implementation of the procedure, the following steps:
a. preparing a sol-gel of a silicon or titanium alcoxide through hydrolysis in the presence of a polar solvent,
b. mixing of sol-gel with a solution containing a surfactant (ionic, preferably cetyl-trimethyl-ammonium-bromide or non-ionic, preferably a block copolymer) dissolved in a polar solvent,
c. synthesizing a film from the mixture above on a suitable substrate by applying one of the following techniques:
   - immersion and extraction at controlled speed ( "dip-coating")
   - coating by centrifugation eg. on a substrate rotating at a controlled speed ( "spin-coating")
   - by spraying ( "spray-coating")
d. densifying the film by drying and / or heat treatment at temperatures above 300 °C, preferably using air at a temperature of about 500 °C;
e. functionalizing the film with a molecule bearing amino groups, in a polar solvent;
f. further functionalizing the film obtained, according to steps d) and e), with a material having affinity for the prion protein through a suitable functional group.

In step f) said material or molecule is preferably selected in the group consisting of: glycosaminoglycans, heparin, polyoxometallates and Congo Red. The functionalized films prepared as described, preferably with a thickness of between 250 and 300 nm, are used, on adequate supports, for the detection of contamination from PrPsc in samples consisting of biological fluids and / or homogenates of solid tissues taken from animals which are potential carriers of the prion infection.

Said detection is made with various detection methods, for example FTIR (Fourier Transformed infrared spectroscopy) or NMR (nuclear magnetic resonance), or through XRD (Powder X ray diffraction), SPR (surface plasmon resonance), UV-Vis (Ultra Violet-Visible), HP (Cyclic Voltammetry) (Bonhomme et al, 2007, Acc. Chem. Res. 2007, 40, 738-746; Kang et al, 2006, Chem Commun (Change)., 28; 2998-3000).

According to a preferred aspect, the assessment of the presence of the pathological prion protein isoform by the method of the invention, namely the assessment of contamination of a sample, is confirmed by reference standard procedures, according to the EC Regulation No. 260/2005, February 16, 2005, such as the Western Blotting rapid test in accordance with the procedure approved by the EU.

Given the substantial structural homology between different "strains" of prions in various pathological forms, this invention is applicable in the revelation of TSEs in general both in animal species and in humans, and in the revelation of other human neurodegenerative diseases caused by accumulation of amyloid substance generated as a result of structural changes, such as Alzheimer's disease.

However, the present invention is particularly suited to identify contamination in biological fluids in fact, said fluids are potential vehicles of illness, but their contamination is so low and unhomogeneous that the systems currently validated for other tissues are not useful.

Indeed, among the "routine tests" to assess the presence of modified prion protein (PrPsc) in the tissues of mammals, developed by various research laboratories and companies and validated according to the criteria set by the European Union, no one is able to detect the presence of PrPsc in biological fluids (blood, milk, urine) and useful for early diagnosis.

Currently, a technique widely used for the analysis of prion protein is Western Blotting (WB) which consists of a immunodiagnostic method associated with the chemiluminescence identification (ECL - Enhanced Chemiluminescence) characterized by high sensitivity and that can ensure the detection of small quantities of specific antigens.

Other techniques developed with the immunodiagnostic approach ELISA, have proven, during practical tests, to be only useful for the detection of prions in tissues containing PrPsc concentrations high enough and - above all - very homogeneous, but not for "in vivo" diagnostic based on the analysis of biological fluids where the PrPsc may be present at very low concentrations and, above all, non homogenously distributed in the liquid.

The usefulness of the present invention is, in particular, to assess the BSE risk in the ovicaprine population, increasing the possibility of diagnosing Scrapie, which can hide BSE. Currently, the European Community has promulgated detailed provisions (EC Regulation No. 99/2001) providing for implementation of rapid tests to determine TSEs positivity on a representative sample in the ovicaprine population, which are always and still implemented on tissues removed from sacrificed animals.

Even in this specific case, the possibility according to the present invention to use huge volumes of organic fluid (even some orders of magnitude higher than those that can be used for standard tests) makes all potential diagnostic applications possible on biological liquids (blood, plasma, urine) and in the application for foods control (useful for both animal and human), where the sample is, for example, milk.

The detection method may comprise a possible treatment of the sample if this is a complex matrix, such as a homogenate tissue, milk, or plasma.

For example, the sample may be enzymatically pre-digested with a proteinase, preferably with proteinase K. So it can be diluted in the suitable buffer, for example PBS and put together with the support coated with the nanostructured material film according to the invention, for a period ranging from several minutes to several hours at a temperature of between +4°C and +30°C, preferably maintained in mild agitation.

As seen above the material that forms the film is selected in the group consisting of: oxides of transition metals, mixed oxides of transition metals and their mixtures. Furthermore, the material is in the mesoporous form with porosity of between 2 and 50 nm and is preferably selected in the group consisting of: silicon oxide, titanium oxide, mixed oxides of silicon and titanium and / or silicon and / or zirconium (SiO₂ - ZrO₂, SiO₂-TiO₂). The sample is then dried and "read" in accordance with the best suited or available analytical method: then it is compared with a film made with the same procedure treated with a sample where there is the normal isoform of the prion protein.

Conveniently, the positive sample can be confirmed by means of the gold standard Western Blot assay.

In the following Experimental Part some examples of preparing the materials in accordance with this invention and the results obtained with different unknown samples and the relative positive and negative samples are presented. These examples do not limit the present invention.

### EXPERIMENTAL PART

### Example 1. Preparation of a hybrid organic-inorganic material for the capturing of the pathological prion protein.

By applying the procedure according to the invention, the TEOS (tetraethyl orthosilicate) is converted into silicon dioxide in the presence of water:

Si(OC₂H₅)₄ + 2 H₂O → SiO₂ + 4 C₂H₅OH

The hydrolysis in the presence of water is an example of a sol-gel process, which gives ethanol as reagent waste. Acids and bases act as catalysts for this reaction by determining the speed of conversion into SiO₂.

In a typical preparation, the TEOS is hydrolyzed in acidic conditions, using hydrochloric acid, water for hydrolysis and ethanol as a solvent. This sol, is used to deposit a thin film. The material is then dried at 60 °C for one hour and then heat-treated to 500 °C. The film is then immersed into a solution of THF to which 3-aminopropyltriethoxysilane (APTES) or 3-aminoethyl-aminopropyltriethoxysilane (AEAPTES) was added at room temperature. With the solution prepared under this procedure, films can be laid on glassy substrates, ITO (Indium tin oxide)-covered glassy substrates, silicon and polymeric substrates by means of spin-coating, dip-coating and spray-coating techniques.

### Example 2. Preparation of a hybrid organic-inorganic mesoporous material whose surface is functionalized with POMs.

A sol precursor was prepared in accordance with the procedure described in example 1, using TEOS or other types of alcoxide precursors. To this solution the surfactant Pluronic 127 dissolved in ethanol, was added. The solution was then immediately used to deposit films on silicon or quartz substrates. The film was then treated at 60 °C for one hour, then at temperatures between 250 and 500 °C to eliminate the surfactant. The 3-aminopropyltriethoxysilane (APTES) or 3-aminoethyl-aminopropyltriethoxysilane (AEAPTES) were then dissolved in ethanol and the film immersed into the solution. This process of amines-anchoring was followed by POMs anchoring. In this example, the sodium salt of the phosphotungstic acid (NaPTA), known in the field of prions science, was used. To anchor the POM on the surface of the film the following steps have been performed: the mesoporous silica film functionalized with APTES was immersed in 15 cc of a solution containing NaPTA dissolved in sulphuric acid [NaPTA (5 mmol /L): 2.8 g NaPTA in 200ml H₂SO₄ (1 N)]. The film was immersed in a beaker and then left in the container closed for 17 hours. After this period, films were removed, washed with ethanol and put into oven at 100 °C for 60 min.

Other examples of preparation / functionalization of the films were made using various substances for anchoring the film, such as Branched Polyethylenimine [H(NHCH₂CH₂) nNH₂ CAS Number: 9002-98-6 (Aldrich)] and Polyethylenimine [(C₂H₅N) n, CAS Number: 25987-06-8 (Aldrich)].

Figure 4 shows the differences in absorbance in the range 1000 to 700 of the various stages of preparation of mesoporous films measured through FITR. Therefore the FTIR can be used to check if the functionalization of the film with different functional groups has occurred.

Various tests were carried out to confirm the ability of mesoporous films functionalized and not functionalized according to the method described in the example above, or to a similar method, to capture / interact with the prion protein in a selective and detectable way between the two forms PrPc and PrPsc, to permit, by using an appropriate detection procedure, to discriminate between them and therefore to set up capture and / or diagnostic systems starting from animal solid tissues homogenates (eg. brain material) and, in parallel, biological fluids (eg. milk and blood). The development of these tests is of particular interest because at present there are no tests able to detect the contamination by prion protein in fluids, but only in tissues derived from biopsies.

There were then carried out validation test of these capturing systems testing the film ability to capture of that ability to capture and discriminate between PrPc and PrPsc, so there were used experimentally known samples, screened by the Western Blot (Prionics Check Western Prod. No. 12000) as a reference test (considered a " gold standard ") able to highlight the presence of different PrPc and PrPsc in the samples.

### Example 3. Results obtained by mesoporous film functionalized with APTES: different interaction with the IR radiation.

Figure 3 shows the FTIR spectra obtained incubating mesoporous films functionalized with APTES in Scrapie positive (S3) and negative (C3) brain homogenate samples. The spectrum has been estimated in the 3800-400 W cm⁻¹ range, divided into the 3800-3300, 1750-1350 and 1000-400 W cm⁻¹ intervals. The area under each curve provides a good estimation of the of the perturbation extent caused by two different samples (C3 and S3): in particular, the area under the spectra produced by S3 (positive homogenate samples) is regularly higher compared to that of the C3 samples (negative homogenate samples) in each of the three selected intervals, in the different samples tested. The measured absorbance indicates the extent of interaction with the IR radiation of various chemical species present in the noted system, both at the surface level and in depth in the film.

### Example 4. Functionalization of the film through binders of prion protein (post-functionalization).

The post-functionalization of the film, made with different compounds, helps to improve the reproducibility of the interaction phenomena between the film and samples containing one of the two PrPc / PrPsc isoforms, as it is confirmed by the results shown either in Figure 5 and Figure 6, obtained by incubating functionalized films (in this example, anchoring POMs molecules, according to the procedure presented in the Example 2) in positive and negative homogenate of samples.

In Figure 5, the brain homogenate samples of sheep, each of which divided into three replicas, were marked with the initials F2C3 (negatives) and F2S3 (positives). In Figure 6, the brain homogenate samples came from either clinically Scrapie positive (S2) and clinically negative sheep (C2). The figures show absolutely similar results both within the same subject (Figure 5) and between different subjects (Figure 6).

The spectra were obtained in a range of particular significance: 3800 - 3200 W cm^{- 1} (area where the absorption frequency of the OH- groups is found and indicating a protein-water molecular interaction). It is possible to see the clear separation of the spectra arising from the negative samples (with absorbance values above the "white" F2C0) from the spectra derived from positive samples (with absorbance values systematically lower than the "white" F2C0).

The spectra shown in Figure 5 (panel A) show that the values of absorbance produced by positive samples are generally lower than those produced by the negative samples. More specifically, in Table 1 where he calculated the average area under the curve of the positive values spectra, it is shown that this value is significantly lower than that of positive samples. This discrimination between positive and negative is further confirmed by the graph of cumulative distributions (Figure 5, panel B).

**Table 1**

| **AREA** | | Norm. Cumul. Distrib. | | | Norm. Cumul. Distrib. |
|---|---|---|---|---|---|
| **Positives** | | | **Negatives** | | |
| | 1760.00 | 0.874226 | | 4500.00 | 0.867885 |
| | 392.00 | 0.324537 | | 1400.00 | 0.38096 |
| | 190.00 | 0.24461 | | 285.00 | 0.207969 |
| Mean value | 780.6667 | | Mean value | 2061.667 | |

These results suggest that the mesoporous film system added with a binder such as a POM (NaPTA as a mere example), interacts differently with biological samples containing PrPsc (F2S3), with β sheets structures, already caught on the surface system, compared with samples containing only PrPc (F2C3) (with prevailing α-helix structure), where the capturing prevails in depth in the film and it is associated with an increase in absorbance values.

The consequence of functionalization that emerges is, therefore, to produce a very dissimilar catching in case of PrPc or PrPsc, revealed by constantly very different absorbance values.

### Example 5. Effect of an enzymatic digestion of the samples captured from the film.

In the prion protein diagnostic procedures validated by the European Union for the purpose of food control and / or used for research in laboratories, the Proteinase K is used to exploit the process of differential PrPc digestion (while PrPsc is resistant to Proteinase K) in order to highlight the differences of the presence of two molecular isoforms in the samples.

As shown in the examples above, the selective capturing of two isoforms by functionalized films makes unnecessary the digestion of the sample, confirming the selective capture of two PrPc / PrPsc isoforms by the film.

As shown in Figure 7, the digestion procedure was applied to 3 samples of Scrapie-positive brain homogenates and to 3 samples of Scrapie-negative brain homogenates, after the film functionalization and by incubation of the film in the homogenate samples (positive or negative) and then dried in accordance with the procedures provided for in this invention. The product thus obtained is subjected to a second incubation period of about 1 hour (or other appropriate times) in a solution containing Proteinase K at convenient concentrations, so that the protein "captured" by the film undergoes to an enzymatic reduction - as reported by the state of knowledge - is most intense in the case of the PrPc isoform than for the PrPsc (the latter is partially resistant to enzymatic digestion).

The films thus obtained were read at the FTIR. The average absorbance level of the 3 negative samples (named C3R1, C3R2, C3R3) decreased significantly more than that of the 3 positive samples (named S3R1, S3R2, S3R3): the results are presented in Table 2, showing in detail the changes in absorbance for the samples between "before" and "after" digestion as described above, recorded as a reduction of the "peak" value in the three main ranges of the spectrum (precisely at 3450 cm⁻¹, 1520 cm⁻¹, 740 cm⁻¹) (see Figure 7, panels A, B, C respectively).

Table 2. Variations of absorbance for the samples, between "before" and "after" digestion as described above, recorded as a reduction of the "peak" value in the three main ranges of the spectrum (precisely at 3450 cm⁻¹, 1520 cm⁻¹, 740 cm⁻¹).

| | **WN cm⁻¹ 3450** | | **WN cm⁻¹ 1520** | | **WN cm⁻¹ 740** | |
|---|---|---|---|---|---|---|
| **Peak** | C3R1:11/35 | S3R1:4/4 | C3R1:4/15 | S3R1:6/10 | C3R1:20/35 | S3R1:2.5/2.5 |
| **Fall** | C3R2:2/2.5 | S3R2:2/3 | C3R2:5/10 | S3R2:3/5 | C3R2:19/17 | S3R2:8/10 |
| **F3/F2** | C3R3:0/13 | S3R3:0/15 | C3R3:6/15 | S3R3:12/12 | C3R3:8/35 | S3R3: 18/28 |
| | **Δm = 12.5** | **Δm = 5.3** | **Δm = 8.3** | **Δm = 2.0** | **Δm = 13.3** | **Δm = 7.3** |

Evidence indicates that the range 3800-3200 WN cm⁻¹ is the most significant to reveal the selective presence of PrPsc on the film.

### Example 6. Selective capture of PrPc / PrPsc in the "milk" with the devices of the invention.

The method object of this invention was used for the detection of PrPc / PrPsc in the milk of sheep through the use of functionalized mesoporous films (MS-APTES-POM). The results were positive, despite the difficulty to control with any reference method the actual level of prion protein present in this liquid and likely the very low concentration of PrPsc in milk.

Figure 8 shows the results of a test carried out by entering PrPsc in the milk of a healthy animal through inoculation ("spiking") of brain homogenate from an animal suffering from Scrapie, as a positive control.

The injection was carried out by adding into each 50 mL of milk sample, a volume of 5 mL of an inoculum obtained by thawing a 10% positive brain homogenate (prepared long before by a laboratory approved for routine checks and kept in freezer) and making a further 1:10 dilution in an appropriate buffer.

The FTIR spectrum of the sample relative to this subject (spike milk) is compared to the spectra obtained from milk samples from various subjects, of whom it cannot be excluded the presence of PrPc / PrPsc in milk, such as Scrapie-positive lactating sheep. A sample (pos. milk 1) presents a spectrum that is very similar to that definitely "positive" (spike milk); the subject of origin of that sample (pos. milk 1) had actually particularly severe clinical Scrapie conditions, worsen by a clinical condition of chronic mastitis. In this subject it was also detected by independent test with the official test of Western Blot the presence of PrPsc in breast tissue, instead absent in the tissues of two other subjects.

### Example 7. Selective capture of PrPc / PrPsc in plasma with the devices of the invention.

The method object of this invention was also used to selectively capture PrPc /PrPsc in the plasma of sheep. In this example, mesoporous films simply functionalized with amines (MS-APTES) were used.

Figure 9 will compare the plasma of clinically positive subjects with those from clinically Scrapie-negative subjects. The results showed a good discrimination between values obtained from two groups of animals, in agreement with what proven with the samples showed in Figure 3. It is noted that, in the considered range, samples from negative subjects produce absorbance curves lower than that produced by the positives, in agreement with what revealed in Figure 3 and as evidenced by the interpolation lines in Figure 9, panel B. In the table 3 below is reported the elaboration of what appears in Figure 9, panels A and B.

**TABLE 3**

| **HEIGHTS** | Norm. Cumul. Distrib. | | | Norm. Cumul. Distrib. | |
|---|---|---|---|---|---|
| Positives | | | Negatives | | |
| | 37.0000 | 0.151766 | | 26.0000 | 0.127853 |
| | 43.0000 | 0.52413 | | 39.0000 | 0.652445 |
| | 48.0000 | 0.833569 | | 42.0000 | 0.771764 |
| Mean value | 42.66667 | | Mean value | 35.66667 | |
| Std. Dev. | 5.507571 | | Std. Dev. | 8.504901 | |

### Example 8. Validation of the selective capturing system.

To validate the ability of the system to selectively capture the two isoforms PrPc and PrPsc of the prion protein, "blind" experiments were carried out by using both positive (ie. containing PrPsc) and negative (ie. containing PrPc only) brain tissue samples already tested by reference "gold standard" procedure with the "immunoblotting" test.

These samples were referred to as "Brain +" and "Brain -"; each sample was divided into 3 replicas (R1, R2, R3), prepared "blinded". These samples (replicas) were tested by Western Blotting, while in parallel were incubated with functionalized mesoporous film (always "blinded"). At the end the FTIR spectra obtained by mesoporous films (MS-APTES-POM) were compared with the WB results and the consistency of results was verified.

The obtained results are shown in Figure 10, from which it is possible to notice the correlation between the results produced with the method object of this invention and those obtained by the "gold standard" testing.

### Example 9. NMR micro-imaging of films obtained in accordance with the invention and incubated into brain homogenate samples positive or negative for Scrapie.

The mesoporous films functionalized in accordance with the procedure of this invention can be analyzed by NMR, with various known approaches (Bonhomme et al, 2008, J. Coat. Technol. Res., 5, 1: 117-121). One method (not unique) is to incubate the film into samples (positive or negative) and after drying it, introducing it in the magnetic field for the micro imaging detection system. This system allows to obtain an image that points out - if any - disturbances due to protein/water interactions. Figure 11 shows as an example the result obtained with positive and negative samples, where it is evident a level of perturbation much more intense in the case of positive samples (containing PrPsc) compared to that of negative samples. This result confirms what obtained with the FTIR spectra and what hypothesized about the selective PrPsc capture on the surface of the film.

## Claims

1. Use of a nanostructured material selected in the group consisting of: oxides of a transition metal, mixed oxides of a transition metal and their mixtures, to detect contamination by a pathological isoform of the prion protein (PrPsc) in a sample.

2. The use, according to claim 1, wherein said nanostructured material is in the mesoporous form with porosity comprised from 2 to 50 nm.

3. The use, according to claims 1 and 2, wherein said nanostructured material is selected in the group consisting of: silicon oxide, titanium oxide, mixed oxides of silicon and titanium and / or silicon and / or zirconium (SiO₂-ZrO₂, SiO₂ - TiO₂).

4. The use, according to claim 3, wherein said material is nanostructured silica or titania.

5. The use, according to claim 1, wherein said nanostructured material is in the form of a film or a nanoparticle.

6. The use, according to claim 5, wherein the nanostructured material is in the form of a film.

7. The use, according to claim 6, wherein the film surface is functionalized with amino- groups.

8. The use, according to claim 7, wherein the surface is further functionalized with a molecule having affinity for the prion protein.

9. The use, according to claim 8, wherein the molecule having affinity for the prion protein is selected from the group consisting of: polyoxometallates (POMs), Congo Red, glycosaminoglycans, heparin or mixtures thereof.

10. The use, according to claims 1-9, wherein said detection is carried out with a method selected from the group consisting of: FT-IR (Fourier Transformed Infra-Red), NMR (nuclear magnetic resonance), XRD (Powder X ray diffraction), SPR (surface plasmon resonance), UV-Vis (UltraVioletto-Visible), HP (Cyclic Voltam metry).

11. The use, according to claim 1, wherein the presence of the pathological isoform of the prion protein (PrPsc) in a sample is an indication that the sample is from an animal suffering from a neurodegenerative disease.

12. The use, according to claim 11, wherein said disease is a Transmissible Spongiform Encephalopathy (TSE) selected from the group consisting of: Creutzfeldt-Jakob disease (CJD), youth variant of the Creutzfeldt-Jakob Disease (vCJD), Gerstmann-Straussler-Scheinker (GSS), Fatal Familial Insomnia (FFI), Scrapie, Bovine Spongiform Encephalopathy (BSE), Feline Spongiform Encephalopathy (ESF) and Scrapie-like forms.

13. The use according to claims 10-12 wherein the signal detected from an unknown sample is compared to the signal detected from a sample where the lack of contamination by the pathological isoform is known.

14. The use according to claims 10-13 wherein said detection is carried out by FITR in a wavelength range comprised from: 3800 to 400 W cm⁻¹.

15. The use according to claim 14 wherein said range is selected in the group of wavelength sub-ranges consisting of: 3800-3200 W cm⁻¹, 1750-1350 W cm⁻¹ and 1000-400 W cm⁻¹.

16. The use according to any one of claims 1-15 wherein the sample is a biological sample or a food sample.

17. The use according to claim 16 wherein the biological or food sample is selected from the group consisting of: e tissue homogenate, milk, plasma or other biological liquid.

18. A process for the preparation of supports coated with a nanostructured material wherein said material is a transition metal oxide selected from mesoporous silica and titania, with a porosity comprised from 2 to 50 nm, **characterized** for comprising the following steps:
a. preparing a sol-gel from a silicon or titanium alcoxide through hydrolysis in the presence of a polar solvent,
b. mixing the sol-gel with a solution comprising a surfactant (ion, preferably cetyl-trimethyl-ammonium-bromide or non-ionic, preferably a block copolymer) dissolved in a polar solvent,
c. synthesizing a film using the mixture, on a suitable substrate according to one of the following techniques:
- immersion and extraction at controlled speed ( "dip-coating")
- deposition by centrifugation eg. on a substrate in rotation at a controlled speed ("spin-coating")
- spraying ( "spray-coating")
d. densifying the film by drying and / or heating at temperatures above 300 °C, preferably with air at a temperature of about 500 °C;
e. functionalizing the film with a molecule bearing amino- groups in a polar solvent;
f. further functionalizing the film densified and functionalized with amino-groups, with a material having affinity for the prion protein by a suitable functional group.

19. The process according to claim 18 wherein said material having affinity for the prion protein is selected from the group consisting of: polyoxometallates, Congo Red, glycosaminoglycans and heparin.

20. The process according to claim 19 wherein the material having affinity for the prion protein is a polyoxometallate (POM) preferably NaPTA (sodium salt of the phosphotungstic acid), the suitable functional group is an alcoxide with aminofunctional groups and functionalization happens in a polar solvent.

21. The process according to any one of claims 18-20 wherein the silicon alcoxide in step a) is tetraethoxysilane (TEOS).

22. The process according to any one of claims 18-21 where the polar solvent in steps b) and e) is an alcohol mixable with water or a water-soluble solvent.

23. The process according to claim 22 where the polar solvent is tetrahydrofuran (THF).

24. The process according to any one of claims 18-23 where in step e) the amino group donor is aminopropyltriethoxysilane (APTES) or aminoethyl-aminopropyltriethoxysilane (AEAPTES).

25. A support coated with a mesoporous silica or titania film, functionalized with a material having affinity for the prion protein selected in the group consisting of: POM, Congo Red, heparin, obtainable according to the process of claims 18-24, to detect contamination by the pathological isoform of prion protein (PrPsc) in a sample.

26. A method to detect the presence of the pathological isoform of the prion protein in a sample comprising the process for the preparation of the mesoporous nanostructured silica or titania coated film having a porosity comprised from 2 to 50 nm according to claims 18-24, and where detection of presence of contamination after incubation of an unknown sample is carried out by FTIR.

27. The method according to claim 26 wherein said presence is an indication that the sample comes from an animal suffering from a neurodegenerative disease.

28. The method according to claim 27 wherein said disease is a neurodegenerative Spongiform Transmissible Encephalopathy (TSE) selected from the group consisting of: Creutzfeldt-Jakob disease (CJD), youth variant of the Creutzfeldt-Jakob disease (vCJD), Gerstmann-Sträussler-Scheinker (GSS), Fatal Familial Insomnia (FFI), Scrapie, Bovine Spongiform Encephalopathy (BSE), Feline Spongiform Encephalopathy (ESF) and Scrapie-like forms.

29. The method according to claims 26-28, wherein FTIR detection is carried out in parallel on a support incubated with a sample comprising the pathological isoform of prion protein (PrPsc) and with a sample comprising the normal isoform (PrPc) as evaluated according to standard reference procedures.

30. The method according to claim 29 wherein the spectra obtained are compared to one another for at least one of the following features: intensity of absorbance, area underlying the curve, shape of the curve and / or translation ("shifting") of natural vibration frequency .

31. A method to detect contamination by prion protein in a sample where the support according to claim 25 is used.

32. The method according to claim 31 wherein the sample is biological or is a food and is selected from the group consisting of: plasma, blood or milk.
